(19) Europäisches Patentamt
European Patent Office
Office européen des brevets

(11) Veröffentlichungsnummer: **0 081 162**
**A2**

# (12) EUROPÄISCHE PATENTANMELDUNG

(21) Anmeldenummer: **82110946.9**

(22) Anmeldetag: **26.11.82**

(51) Int. Cl.³: **C 07 D 207/456**
A 01 N 43/36

(30) Priorität: **02.12.81 DE 3147638**

(43) Veröffentlichungstag der Anmeldung:
**15.06.83 Patentblatt 83/24**

(84) Benannte Vertragsstaaten:
**AT CH DE FR IT LI**

(71) Anmelder: **HOECHST AKTIENGESELLSCHAFT**
**Postfach 80 03 20**
**D-6230 Frankfurt/Main 80(DE)**

(72) Erfinder: **Mildenberger, Hilmar, Dr.**
**Fasanenstrasse 24**
**D-6233 Kelkheim (Taunus)(DE)**

(72) Erfinder: **Gerber, Hans-Gerd, Dr.**
**Lortzingstrasse 67**
**D-4010 Hilden(DE)**

(72) Erfinder: **Matterstock, Karl, Dr.**
**Lessingstrasse 38**
**D-6238 Hofheim am Taunus(DE)**

(72) Erfinder: **Sachse, Burkhard, Dr.**
**An der Ziegelei 30**
**D-6233 Kelkheim (Taunus)(DE)**

(72) Erfinder: **Hartz, Peter, Dr.**
**An der Ziegelei 28**
**D-6233 Kelkheim (Taunus)(DE)**

(54) **Fungizide und bakterizide 2-Dihalogenmethylenpyrrolinone.**

(57) 2-Dihalogenmethylen-3-alkoxycarbonyl-3-pyrrolin-5-one der allgemeinen Formel I,

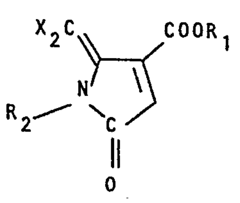

(I)

worin
$R_1$ = Wasserstoff oder Alkyl, $R_2$ = Wasserstoff, Alkyl, Alkoxycarbonylmethyl, Cyclohexyl, Benzyl oder ggf. substituiertes Phenyl und X = Halogen bedeutet lassen sich vorteilhaft zur Bekämpfung von Pilzen und Bakterien im landwirtschaftlichen und technischen Bereich einsetzen.

EP 0 081 162 A2

0081162

HOECHST AKTIENGESELLSCHAFT HOE 81/F 319 Dr.AU/Wa

# Fungizide und bakterizide 2-Dihalogenmethylenpyrrolinone

Gegenstand der vorliegenden Erfindung sind neue, fungizide und bakterizide 2-Dihalogenmethylen-3-alkoxycarbonyl-3-pyrrolin-5-one, Verfahren zu ihrer Herstellung und ihre Verwendung als Schädlingsbekämpfungsmittel, insbesondere im Pflanzenschutz.

Die erfindungsgemäßen Verbindungen besitzen die allgemeine Formel I,

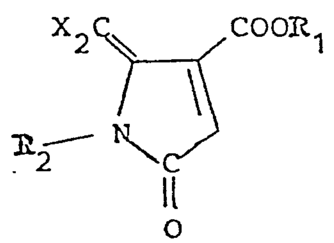

(I)

worin

$R_1$ = Wasserstoff oder $(C_1-C_4)$-Alkyl,

$R_2$ = Wasserstoff, $(C_1-C_4)$-Alkyl, $(C_1-C_4)$-Alkoxycarbonylmethyl, Cyclohexyl, Benzyl, Phenyl oder durch $(C_1-C_4)$-Alkyl, Halogen, Nitro, $(C_1-C_4)$-Alkoxy, $(C_1-C_4)$-Alkoxycarbonyl oder Trihalogenmethyl substituiertes Phenyl und

X = Halogen bedeuten.

Bevorzugte Reste in Formel I sind z.B.

$R_1$ = $(C_1-C_4)$-Alkyl,

$R_2$ = Methyl, Methoxycarbonylmethyl, Phenyl, 2,4-Dichlorphenyl, 3-Trifluormethylphenyl, Benzyl, 2,6-Dimethylphenyl, 2-Chlor-4-trifluormethylphenyl, 3-Chlor-4-methylphenyl, 3,5-bis-Trifluormethylphenyl, 2,3-Dimethylphenyl, 4-Ethoxycarbonylphenyl, 4-Chlorphenyl, 4-Methoxyphenyl, 2-Methoxyphenyl oder 4-Bromphenyl, und

X = Cl.

Besonders bevorzugt hiervon sind Verbindungen der Formel I mit $R_2$ = Phenyl.

Folgende Verbindungen stellen eine beispielhafte Auswahl an erfindungsgemäßen Verbindungen der Formel I dar:

2-Dichlormethylen-3-methoxycarbonyl-3-pyrrolin-5-on,
1-Methyl-2-dichlormethylen-3-methoxycarbonyl-3-pyrrolin-5-on,
1-Benzyl-2-dichlormethylen-3-ethoxycarbonyl-3-pyrrolin-5-on,
1-(4-Chlorbenzyl)-2-dichlormethylen-3-methoxycarbonyl-3-pyrrolin-5-on,
1-(2,4-Dichlorbenzyl-2-dichlormethylen-3-ethoxycarbonyl-3-pyrrolin-5-on,
1-Cyclohexyl-2-dichlormethylen-3-methoxycarbonyl-3-pyrrolin-5-on,
1-Phenyl-2-dichlormethylen-3-methoxycarbonyl-3-pyrrolin-5-on,
1-(4-Methylphenyl)-2-dichlormethylen-3-ethoxycarbonyl-3-pyrrolin-5-on,
1-(2,6-Dimethylphenyl)-2-dichlormethylen-3-methoxycarbonyl-3-pyrrolin-5-on,
1-(2-Methyl-6-ethylphenyl)-2-dichlormethylen-3-methoxycarbonyl-3-pyrrolin-5-on,
1-(2,3-Dimethylphenyl)-2-dichlormethylen-3-methoxycarbonyl-3-pyrrolin-5-on,
1-(2,6-Diethylphenyl)-2-dichlormethylen-3-ethoxycarbonyl-3-pyrrolin-5-on,
1-(4-Ethoxycarbonylphenyl)-2-dichlormethylen-3-methoxycarbonyl-3-pyrrolin-5-on,
1-(4-Chlorphenyl)-2-dichlormethylen-3-methoxycarbonyl-3-pyrrolin-5-on,
1-(2,4-Dichlorphenyl)-2-dichlormethylen-3-methoxycarbonyl-3-pyrrolin-5-on,
1-(2,6-Dichlorphenyl)-2-dichlormethylen-3-methoxycarbonyl-3-pyrrolin-5-on,
1-(3,5-Dichlorphenyl)-2-dichlormethylen-3-methoxycarbonyl-3-pyrrolin-5-on,

1-(4-Bromphenyl)-2-dichlormethylen-3-methoxycarbonyl-3-pyrrolin-5-on,
1-(4-Fluorphenyl)-2-dichlormethylen-3-methoxycarbonyl-3-pyrrolin-5-on,
1-(4-Methoxyphenyl)-2-dichlormethylen-3-ethoxycarbonyl-3-pyrrolin-5-on,
1-(2-Methoxyphenyl)-2-dichlormethylen-3-methoxycarbonyl-3-pyrrolin-5-on,
1-(3-Trifluormethylphenyl)-2-dichlormethylen-3-methoxycarbonyl-3-pyrrolin-5-on,
1-(3,5-bis-Trifluormethyl)-2-dichlormethylen-3-methoxycarbonyl-3-pyrrolin-5-on,
1-(2-Chlor-4-trifluormethylphenyl)-2-dichlormethylen-3-methoxycarbonyl-3-pyrrolin-5-on,
1-(3-Chlor-4-methylphenyl)-2-dichlormethylen-3-methoxycarbonyl-3-pyrrolin-5-on,
1-(3-Chlor-6-methylphenyl)-2-dichlormethylen-3-ethoxycarbonyl-3-pyrrolin-5-on,
1-(4-Nitrophenyl)-2-dichlormethylen-3-methoxycarbonyl-3-pyrrolin-5-on,
1-(2-Chlor-6-methylphenyl)-2-dichlormethylen-3-methoxycarbonyl-3-pyrrolin-5-on.

Gegenstand der Erfindung ist weiterhin ein Verfahren zur Herstellung von Verbindungen der Formel I, das dadurch gekennzeichnet ist, daß man aus 2-Dihalogenmethylen-3-halogen-3-alkoxycarbonylpyrrolidin-5-onen der allgemeinen Formel II,

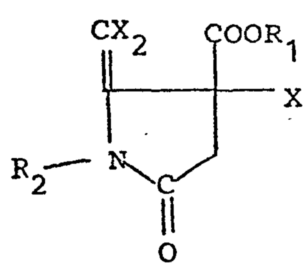

(II)

worin $R_1$, $R_2$ und X die Bedeutungen wie in Formel I haben, durch Umsetzung mit basischen Verbindungen Halogenwasserstoff abspaltet, gegebenenfalls in inerten Lösungsmitteln.

Die Umsetzungstemperatur ist nicht kritisch und liegt bevorzugt im Bereich zwischen -5 und 80°C, insbesondere zwischen 0 und 60°C.

Die Verbindungen der allgemeinen Formel II können gemäß den Angaben in der DE-OS 29 28 305 hergestellt werden.

Als basische Verbindungen kommen anorganische oder organische basische Verbindungen in Frage, vorzugsweise tertiäre organische Basen, wie z.B. Trialkylamine, Pyridin, Alkali-oder Erdalkalicarbonate.Es sind für die erfindungsgemäße Umsetzung mindestens stöchiometrische Mengen an basischen Verbindungen erforderlich. Die Durchführung der Umsetzung in inerten Lösungsmitteln ist bevorzugt. Als inerte Lösungsmittel kommen solche Verbindungen in Frage, die sich unter den Reaktionsbedingungen gegenüber den Reaktanten inert verhalten,z.B. aprotische organische Lösungsmittel. Bevorzugte inerte Lösungsmittel sind z.B. aliphatische und aromatische Kohlenwasserstoffe, beispielsweise Cyclohexan, Benzol, Toluol, Xylol oder deren Gemische.

Die Verbindungen der Formel I sind teilweise im Reaktionsgemisch schwer löslich und können dann durch Absaugen gewonnen werden, oder sie fallen nach dem Abdampfen des verwendeten Lösungsmittels als Öle an, die beim Verreiben mit Ether oder Benzin kristallisieren. Durch Umkristallisieren oder durch Chromatographie können sie weiter gereinigt werden.

Die erfindungsgemäßen Verbindungen der Formel I weisen überraschenderweise eine relativ breite biozide Wirkung

gegen Pilze und Bakterien auf. Sie sind besonders wirksam gegen phytopathogene Pilze, wie z.B. Botrytis cinerea, Rostpilze, Cercospora beticola, Cladosporium fulvum, Venturia inaequalis, Piricularia oryzae und Rhizoctonia solani. Eine hervorragende Wirkung zeigen die erfindungsgemäßen Verbindungen gegen die der Klasse der Phycomycetes angehörenden Oomyceten, wie z.B. Phytophtora, Peronospora, Pseudoperonospora, Plasmopara und Pythium, insbesondere gegen Plasmopara.

Die Verbindungen lassen sich ferner zur Bekämpfung von nicht-phytopathogenen Pilzen und Bakterien, die auf technischen Substraten wachsen und diese abbauen oder zerstören können, einsetzen. Sie erfassen u.a. Aureobasidium pullulans, Ulocladium consortiale, Aspergillus niger, Penicillium funiculosum, Poria monticola und Coniophora puteana. Ebenso werden Bakterienarten wie z.B. Bacillus subtilis und Areobacter aerogenes und Escherichia coli durch die beanspruchten Verbindungen der Formel I in ihrem Wachstum gehemmt.

Gegenstand der Erfindung sind daher auch fungizide und bakterizide Mittel, die gekennzeichnet sind durch einen Gehalt an einer Verbindung der Formel I.

Die fungiziden und bakteriziden Mittel lassen sich in üblicher Weise, z.B. als Stäube, Spritzpulver, Beizmittel, Dispersionen, Lösungen oder Emulsionskonzentrate formulieren. Der Gehalt an Wirkstoff der Formel I beträgt in den erfindungsgemäßen Mitteln im allgemeinen etwa 2 bis 95 Gew.-%, vorzugsweise 10 bis 90 Gew.-%, wobei der Rest zu 100 Gew.-% aus üblichen Formulierungshilfsmitteln, wie Haft-, Netz-, Dispergier-, Emulgier-, Penetrations-, Lösungsmitteln, Füll- und Trägerstoffen besteht.

Die beanspruchten Verbindungen der Formel I eignen sich

auch für den Einsatz im technischen bzw. im chemisch-technischen Bereich, beispielsweise in Holzschutzmitteln, auf dem Anstrichfarbensektor oder als Konservierungsmittel, z.B. in Kühlschmiermitteln für die Metallbearbeitung.

Die Erfindung wird durch die nachfolgenden Beispiele näher erläutert.

## A. Herstellungsbeispiele

### Beispiel 1

36,9 g (0,1 Mol) 1-(4-Chlorphenyl)-2-dichlormethylen-3-chlor-3-methoxycarbonyl-pyrrolidin-5-on werden in 200 ml Toluol gelöst. Bei 5 bis 10°C werden 13 g Triethylamin, gelöst in 50 ml Toluol unter Rühren zugetropft. Anschließend wird 2 Stunden bei Raumtemperatur nachgerührt. Das Reaktionsgemisch wird zweimal mit Wasser gewaschen, über $Na_2SO_4$ getrocknet und unter Vakuum das Lösungsmittel abdestilliert. Als Rückstand verbleiben 34 g eines hellbraunen Öls, das beim Verreiben mit Isopropylether kristallisiert. Man saugt ab und erhält 26 g (entsprechend 79 % der Theorie) 1-(4-Chlorphenyl)-2-dichlormethylen-3-methoxycarbonyl-3-pyrrolin-5-on. Fp.: 108°C.

### Beispiele 2-13

In der nachstehenden Tabelle 1 sind die Reste $R_1$, $R_2$ und X in Formel I der nach den Beispielen 2 bis 13 aus den entsprechenden Verbindungen der Formel II durch Halogenwasserstoffabspaltung analog dem Beispiel 1 hergestellten Verbindungen der Formel I sowie deren Schmelzpunkte zusammengefaßt aufgeführt.

TABELLE 1

Formel I:

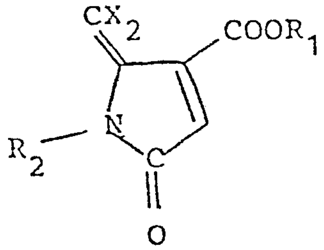

| Beispiel Nr. | $R_1$ | $R_2$ | X | Fp. [°C] |
|---|---|---|---|---|
| 2 | $-CH_3$ | 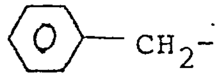 | Cl | 98 |
| 3 | $-CH_3$ | 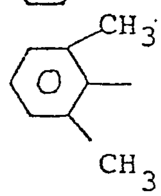 | Cl | 112 |
| 4 | $-CH_3$ | 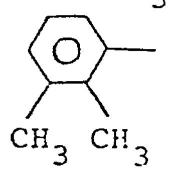 | Cl | 109 |
| 5 | $-CH_3$ | 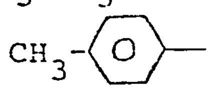 | Cl | 112 |
| 6 | $-CH_3$ | 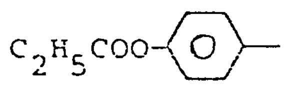 | Cl | 106 |
| 7 | $-CH_3$ | 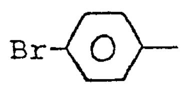 | Cl | 133 |
| 8 | $-CH_3$ | 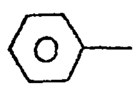 | Cl | 112 |
| 9 | $-CH_3$ | 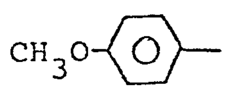 | Cl | 151 |
| 10 | $-CH_3$ | 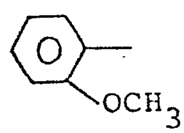 | Cl | 112 |
| 11 | $-CH_3$ | 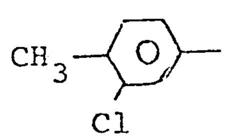 | Cl | 106 |
| 12 | $-CH_3$ | 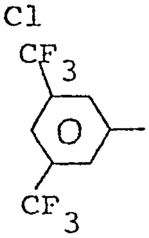 | Cl | 144 |
| 13 | $-C_2H_5$ | 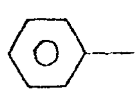 | Cl | 102 |

## B. Formulierungsbeispiele

### Beispiel A

Ein in Wasser leicht dispergierbares, benetzbares Pulver wird erhalten, indem man
25 Gewichtsteile Wirkstoff der Formel I,
64 Gew.-Teile kaolinhaltigen Quarz als Inertstoff,
10 Gew.-Teile ligninsulfonsaures Natrium und
1 Gew.-Teil oleoylmethyltaurinsaures Natrium als Netz- und Dispergiermittel
mischt und in einer Stiftmühle mahlt.

### Beispiel B

Ein Stäubemittel, das sich zur Anwendung als Pilzvertilgungsmittel gut eignet, wird erhalten, indem man
10 Gew.-Teile Wirkstoff der Formel I und
90 Gew.-Teile Talkum als Inertstoff
mischt und in einer Schlagmühle zerkleinert.

### Beispiel C

Ein emulgierbares Konzentrat wird erhalten durch Vermischen von
15 Gew.-Teilen Wirkstoff der Formel I,
75 Gew.-Teilen Cyclohexanon als Lösungsmittel und
10 Gew.-Teilen oxethyliertes Nonylphenol (10 AeO) als Emulgator.

### Beispiel D

Ein Granulat wird erhalten, indem man 2-15 Gew.-Teile Wirkstoff der Formel I in Toluollösung an einem inerten Granulatträgermaterial der gewünschten Korngröße, wie z Attapulgut, Bimsgranulat oder Quarzsand adsorbiert und Lösungsmittel anschließend verdunsten läßt.

## C. Biologische Beispiele

Die erfindungsgemäßen Verbindungen der Formel I zeichnen sich durch eine hervorragende fungizide und bakterizide Wirkung aus, wie die nachstehenden Beispiele I bis IV zeigen.

### Beispiel I

**Wirkung gegen Phytophthora infestans auf Solanum lycopersicum**

Tomatenpflanzen (Solanum lycopersicum) der Sorte Rheinlands Ruhm wurden im 3-Blattstadium mit den in der Tabelle I angegebenen Verbindungen der Formel I in Wirkstoffkonzentrationen von jeweils 250, 125 und 60 mg/Liter Spritzbrühe tropfnaß gespritzt.

Nach dem Antrocknen des Spritzbelages wurden die Pflanzen mit einer Zoosporangiensuspension von Phytophthora infestans stark inokuliert und einen Tag lang tropfnaß in eine Klimakammer bei einer Temperatur von 15 °C und einer rel. Luftfeuchtigkeit von 100 % gestellt. Anschließend kamen sie in ein Kühlgewächshaus mit einer Temperatur von 15°C und einer rel. Luftfeuchtigkeit von 85 - 95 %.

Nach einer Inkubationszeit von 7 Tagen wurden die Pflanzen auf Befall mit Phytophthora untersucht. Anzahl und Größe der typischen Phytophthora-Blattflecken dienten als Bewertungsmaßstab für die Wirksamkeit der geprüften Verbindungen. Der Befallsgrad wurde ausgedrückt in % befallener Blattfläche im Vergleich zu unbehandelten, infizierten Kontrollpflanzen (= 100 % Befall). Das Versuchsergebnis ist in der Tabelle I zusammengefaßt wiedergegeben.

Tabelle I

| Verbindung gemäß Beispiel Nr. | % Phytophthora-Befall bei mg Wirkstoff/ Ltr. Spritzbrühe | | |
|---|---|---|---|
| | 250 (mg) | 125 (mg) | 60 (mg) |
| 9 | 0 | 0-3 | 3 |
| 8 | 0 | 0-3 | 5 |
| 3 | 0-3 | 3 | 5 |
| Unbehandelte infiz. Pflanzen | 100 | | |

Beispiel II

Wirkung gegen Plasmopara viticola auf Weinreben

Weinpflanzen, die aus Stecklingen der Plasmoparaanfälligen Sorte Müller-Thurgau gezogen waren, wurden im 4-Blattstadium mit wässrigen Suspensionen der zu prüfenden Verbindungen der Formel I in Anwendungskonzentrationen von jeweils 250, 125 und 60 mg Wirkstoff/Ltr. Spritzbrühe tropfnaß gespritzt.

Nach dem Antrocknen des Spritzbelages wurden die Pflanzen mit einer Zoosporangiensuspension von Plasmopara viticola stark inokuliert und tropfnaß in eine Klimakammer bei einer Temperatur von 20°C und einer rel. Luftfeuchtigkeit von 100 % gestellt. Nach 24 Stunden wurden die infizierten Pflanzen der Klimakammer entnommen und in ein Gewächshaus mit einer Temperatur von 23°C und einer rel. Luftfeuchtigkeit von ca. 80-90 % gebracht.

Nach einer Inkubationszeit von 7 Tagen wurden die

Pflanzen angefeuchtet, über Nacht in die Klimakammer gestellt und die Krankheit zum Ausbruch gebracht. Anschließend erfolgte die Befallsauswertung. Anzahl und Größe der Infektionsstellen auf den Blättern der inokulierten und behandelten Pflanzen dienten als Maßstab für die Wirksamkeit der geprüften Verbindungen. Der Befallsgrad wurde in % befallener Blattfläche im Vergleich zu den unbehandelten, infizierten Kontrollpflanzen (= 100 % Befall) ausgedrückt. Das Versuchsergebnis ist in der Tabelle II zusammengefaßt wiedergegeben.

Tabelle II

| Verbindung gemäß Beispiel Nr. | % Plasmopara viticola-Befall bei mg Wirkstoff/Ltr. Spritzbrühe | | |
|---|---|---|---|
| | 250 (mg) | 125 (mg) | 60 (mg) |
| 10 | 0 | 0-3 | 5 |
| 9 | 0-3 | 3 | 3-5 |
| 8 | 0 | 0 | 0 |
| 6 | 0 | 0-3 | 5 |
| 3 | 0 | 0 | 0-3 |
| 2 | 0 | 0 | 0-3 |
| Unbehandelte infiz. Pflanzen | 100 | | |

Beispiel III

Wirkung gegen Bacillus subtilis auf Nähragar

Jeweils 0,02 ml einer Bakteriensuspension von Bacillus subtilis wurden in Petrischalen auf Nährböden (Standard-I-Nähragar für Bakterien) tropfenförmig aufgebracht; dem Agar waren zuvor im flüssigen Zustand die

zu prüfenden Verbindungen der Formel I in den in der Tabelle III angegebenen Konzentrationen zugesetzt worden.

Die mit Bakterien beimpften Agar-Platten wurden nach 4 Tagen ausgewertet; hierbei wurde die Hemmung des Bakterien-Wachstums im Vergleich zur Kontrolle (= beimpfter Agar ohne Wirkstoffzusatz = 0 % Hemmung) bonitiert. Das Ergebnis ist in der Tabelle III zusammengefaßt wiedergegeben.

Tabelle III

| Verbindung gemäß Beispiel Nr. | Hemmung in % von Bacillus subtilis bei mg Wirkstoff/Ltr. Agar | |
|---|---|---|
| | 50 | 10 |
| 11 | 100 | 50 |
| 10 | 100 | 50 |
| 8 | 100 | 50 |
| 1 | 100 | 40 |
| 5 | 100 | 40 |
| 4 | 100 | 50 |
| 3 | 100 | 100 |
| 2 | 100 | 40 |
| unbehandelter infiz. Nähragar | 0 | |

Beispiel IV

Wirkung gegen Ulocladium consortiale auf Nähragar

Jeweils 0,02 ml einer Sporensuspension von Ulocladium consortiale wurden in Petrischalen auf Nährböden (Biomalz-Agar für Pilze) tropfenförmig aufgebracht; dem Agar waren zuvor im flüssigen Zustand die zu prüfenden

Verbindungen der Formel I in den in der Tabelle IV angegebenen Konzentrationen zugesetzt worden. 6 Tage nach der Beimpfung der Agar-Platten wurde der Durchmesser der Pilzkolonien auf dem Agar ausgemessen und die durch die zu prüfenden Verbindungen der Formel I hervorgerufene Wachstumshemmung, ausgedrückt in % und bezogen auf die Kontrolle (= beimpfter Agar ohne Wirkstoffzusatz = 0 % Hemmung), bonitiert.
Das Ergebnis ist in der Tabelle IV zusammengefaßt wiedergegeben.

Tabelle IV

| Verbindung gemäß Beispiel Nr. | Hemmung in % von Ulocladium consortiale bei mg Wirkstoff/Ltr. Agar | | |
|---|---|---|---|
| | 500 | 100 | 50 |
| 5 | 100 | 100 | 50 |
| 2 | 100 | 80 | 80 |
| unbehandelter, infiz. Nähragar | 0 | | |

PATENTANSPRÜCHE:

1. 2-Dihalogenmethylen-3-alkoxycarbonyl-3-pyrrolin-5-one der allgemeinen Formel I,

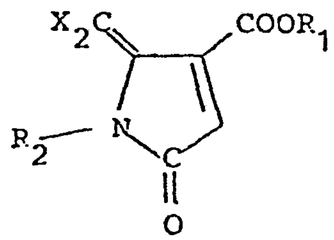

(I)

worin

$R_1$ = Wasserstoff oder $(C_1-C_4)$-Alkyl,

$R_2$ = Wasserstoff, $(C_1-C_4)$-Alkyl, $(C_1-C_4)$-Alkoxycarbonylmethyl, Cyclohexyl, Benzyl, Phenyl oder durch $(C_1-C_4)$-Alkyl, Halogen, Nitro, $(C_1-C_4)$-Alkoxy, $(C_1-C_4)$-Alkoxycarbonyl oder Trihalogenmethyl substituiertes Phenyl und

X = Halogen bedeuten.

2. Verbindungen der Formel I gemäß Anspruch 1, dadurch gekennzeichnet, daß

$R_1$ = $(C_1-C_4)$-Alkyl,

$R_2$ = Methyl, Methoxycarbonylmethyl, Phenyl, 2,4-Dichlorphenyl, 3-Trifluormethylphenyl, Benzyl, 2,6-Dimethylphenyl, 2-Chlor-4-trifluormethylphenyl, 3-Chlor-4-methylphenyl, 3,5-bis-Trifluormethylphenyl, 2,3-Dimethylphenyl, 4-Ethoxycarbonylphenyl, 4-Chlorphenyl, 4-Methoxyphenyl, 2-Methoxyphenyl oder 4-Bromphenyl, und

X = Cl

bedeuten.

3. Verbindungen der Formel I gemäß Anspruch 2, dadurch gekennzeichnet, daß $R_2$ = Phenyl bedeutet.

4. Verfahren zur Herstellung von Verbindungen der Formel I

gemäß Ansprüchen 1 bis 3, dadurch gekennzeichnet, daß man aus 2-Dihalogenmethylen-3-halogen-3-alkoxy-carbonyl-pyrrolidin-5-onen der allgemeinen Formel II,

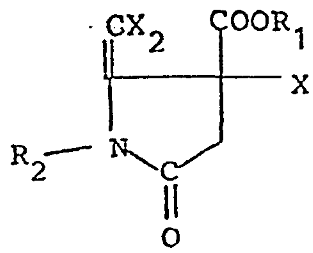

(II)

worin $R_1$, $R_2$ und X die Bedeutungen wie in Formel I haben, durch Umsetzung mit basischen Verbindungen Halogenwasserstoff abspaltet, ggfs. in inerten Lösungsmitteln.

5. Verfahren nach Anspruch 4, dadurch gekennzeichnet, daß man als basische Verbindungen tertiäre organische Basen einsetzt.

6. Schädlingsbekämpfungsmittel, dadurch gekennzeichnet, daß sie eine Verbindung der Formel I gemäß Ansprüchen 1 bis 3 als Wirkstoff und gegebenenfalls Formulierungshilfsmittel enthalten.

7. Schädlingsbekämpfungsmittel, dadurch gekennzeichnet, daß sie 2 bis 95 Gew.-%, vorzugsweise 10 bis 90 Gew.-%, einer Verbindung der Formel I gemäß Ansprüchen 1 bis 3 als Wirkstoff enthalten und der Rest zu 100 Gew.-% aus üblichen Formulierungshilfsmitteln besteht.

8. Verwendung von Verbindungen der Formel I gemäß Ansprüchen 1 bis 3, 6 und 7 zur Schädlingsbekämpfung im Pflanzenschutz.

9. Verwendung von Verbindungen der Formel I gemäß Ansprüchen 1 bis 3, 6 und 7 zur Schädlingsbekämpfung im technischen bzw. chemisch-technischen Bereich.

10. Verfahren zur Bekämpfung von Schadpilzen und Bakterien, dadurch gekennzeichnet, daß man die von ihnen befallenen Flächen, Pflanzen oder Substrate mit einer fungizid und bakterizid wirksamen Menge von Wirkstoffen der Formel I gemäß Ansprüchen 1 bis 3, 6 und 7 behandelt bzw. in Kontakt bringt.

Patentansprüche Österreich

1. Verfahren zur Herstellung von Verbindungen der Formel I

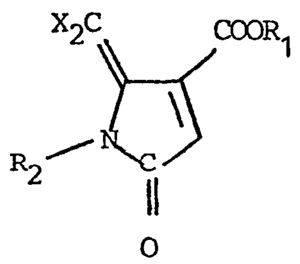

(I)

worin

$R_1$ = Wasserstoff oder $(C_1-C_4)$-Alkyl,

$R_2$ = Wasserstoff, $(C_1-C_4)$-Alkyl, $(C_1-C_4)$-Alkoxycarbonylmethyl, Cyclohexyl, Benzyl, Phenyl oder durch $(C_1-C_4)$-Alkyl, Halogen, Nitro, $(C_1-C_4)$-Alkoxy, $(C_1-C_4)$-Alkoxycarbonyl oder Trihalogenmethyl substituiertes Phenyl und

X = Halogen bedeuten, dadurch gekennzeichnet, daß man aus 2-Dihalogenmethylen-3-halogen-3-alkoxycarbonylpyrrolidin-5-onen der allgemeinen Formel II,

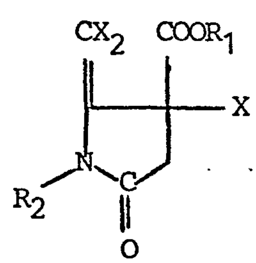

(II)

worin $R_1$, $R_2$ und X die Bedeutungen wie in Formel I haben, durch Umsetzung mit basischen Verbindungen Halogenwasserstoff abspaltet, ggfs. in inerten Lösungsmitteln.

2. Verfahren nach Anspruch 1, dadurch gekennzeichnet, daß man als basische Verbindungen tertiäre organische Basen einsetzt.

3. Schädlingsbekämpfungsmittel, dadurch gekennzeichnet, daß sie eine Verbindung der Formel I gemäß Anspruch 1 als Wirkstoff und gegebenenfalls Formulierungshilfsmittel enthalten.

4. Schädlingsbekämpfungsmittel, dadurch gekennzeichnet, daß sie 2 bis 95 Gew.-%, vorzugsweise 10 bis 90 Gew.-%, einer Verbindung der Formel I gemäß Anspruch 1 als Wirkstoff enthalten und der Rest zu 100 Gew.-% aus üblichen Formulierungshilfsmitteln besteht.

5. Verwendung von Verbindungen der Formel I gemäß Ansprüchen 3 und 4 zur Schädlingsbekämpfung im Pflanzenschutz.

6. Verwendung von Verbindungen der Formel I gemäß Ansprüchen 3 und 4 zur Schädlingsbekämpfung im technischen bzw. im chemisch-technischen Bereich.

7. Verfahren zur Bekämpfung von Schadpilzen und Bakterien, dadurch gekennzeichnet, daß man die von ihnen befallenen Flächen, Pflanzen oder Substrate mit einer fungizid und bakterizid wirksamen Menge von Wirkstoffen der Formel I gemäß Ansprüchen 3 und 4 behandelt bzw. in Kontakt bringt.